# FASCICULE DE BREVET EUROPEEN

(11) **EP 1 868 632 B1**
(45) Date de publication et mention de la délivrance du brevet: **07.09.2016**
(21) Numéro de dépôt: 06743628.7
(22) Date de dépôt: 30.03.2006
(51) Int. Cl.: A61K 38/08, A61Q 17/04, A61Q 19/08, A61K 8/64, A61K 38/16, A61K 38/43, A61K 38/10

(54) **COMPOSITION DERMATOLOGIQUE ET /OU COSMÉTIQUE CONTENANT DES PROTEINES DE LA FAMILLE DES SIRT**
DERMATOLOGISCHE UND/ODER KOSMETISCHE ZUSAMMENSETZUNG MIT PROTEINEN DER SIRT-FAMILIE
DERMATOLOGICAL AND/OR COSMETIC COMPOSITION CONTAINING PROTEINS OF THE SIRT FAMILY

(30) Priorité: 01.04.2005 FR 0503205
(43) Date de publication de la demande: 26.12.2007
(73) Titulaire: Ashland Specialties France, 06410 Biot (FR)
(72) Inventeur: DAL FARRA, Claude, F-06650 Opio (FR); DOMLOGE, Nouha, F-06560 Valbonne (FR); BOTTO, Jean-Marie, F-06560 Valbonne (FR)
(74) Mandataire: Macquet, Christophe
(86) Numéro de dépôt international: PCT/FR2006/000697
(87) Numéro de publication internationale: WO 2006/103354

(56) Documents cités:
- WO-A-03/061681
- WO-A-2005/065667
- WO-A2-2005/002555
- CN-A- 1 339 496
- PORCU M ET AL: "The emerging therapeutic potential of sirtuin-interacting drugs: from cell death to lifespan extension" TRENDS IN PHARMACOLOGICAL SCIENCES, ELSEVIER, HAYWARTH, GB, vol. 26, no. 2, février 2005 (2005-02), pages 94-103, XP004727629 ISSN: 0165-6147
- HEKIMI S ET AL: "Genetics and the specificity of the aging process" SCIENCE, AMERICAN ASSOCIATION FOR THE ADVANCEMENT OF SCIENCE,, US, vol. 299, no. 5611, 28 février 2003 (2003-02-28), pages 1351-1354, XP002343916 ISSN: 0036-8075
- GUARENTE L: "Sir2 links chromatin silencing, metabolism, and aging" GENES AND DEVELOPMENT 01 MAY 2000 UNITED STATES, vol. 14, no. 9, 1 mai 2000 (2000-05-01), pages 1021-1026, XP002421937 ISSN: 0890-9369
- FRYE R A: "PHYLOGENETIC CLASSIFICATION OF PROKARYOTIC AND EUKARYOTIC SIR2-LIKE PROTEINS" BIOCHEMICAL AND BIOPHYSICAL RESEARCH COMMUNICATIONS, ACADEMIC PRESS INC. ORLANDO, FL, US, vol. 273, no. 2, 5 juillet 2000 (2000-07-05), pages 793-798, XP001206835 ISSN: 0006-291X

## Description

La présente invention se situe dans le domaine de la pharmaceutique, et plus particulièrement dans le domaine de la dermatologie et de la cosmétologie. La présente invention concerne des compositions contenant des fragments polypeptidiques ou peptidiques de protéines SIRT, en tant qu'agent actif, seul ou en association avec au moins un autre agent actif, destinées à être utilisées dans les domaines pharmaceutique et/ou dermatologique et/ou cosmétique.

La peau est un organe de revêtement recouvrant la totalité de la surface du corps. C'est un organe vital assurant des fonctions multiples telles que des fonctions sensitives, protectrices vis-à-vis d'agressions externes multiples, immunitaires, métaboliques ou encore thermorégulatrices. Ces rôles sont rendus possibles grâce à une structure complexe qui associe des structures tissulaires variées. La peau, ainsi que les phanères, sont soumis constamment à des nuisances d'origine extérieure qui se manifestent sous différents aspects. Ces agressions peuvent être importantes et elles peuvent menacer l'équilibre et l'aspect de la peau. A titre d'exemple, on peut citer les rayonnements visibles ou ultraviolets, les polluants atmosphériques, les produits chimiques, les stress d'origines mécaniques, les infections virales ou bactériennes...

La peau est notre première barrière contre l'environnement extérieur, cependant cette barrière n'est pas toujours suffisante et ces agressions peuvent avoir de nombreuses conséquences néfastes pour l'organisme, tant au niveau de celui-ci qu'au niveau cellulaire. Les cellules de la peau, en contact direct avec le milieu extérieur, connaissent de nombreux dommages, notamment une altération de leurs macromolécules. On peut citer, par exemple, les dommages occasionnés à l'ADN (tels que des coupures double ou simple brin, des mutations...), aux protéines (carbonylation des protéines...), modifications des acides gras (péroxydation des lipides...) ayant comme conséquence des dommages au niveau des membranes cellulaires et une altération de la barrière cutanée.
Ces altérations ont lieu sur tous types cellulaires cutanés, mais on peut citer en particulier : les kératinocytes, les fibroblastes et les mélanocytes. Ces agressions extérieures résultent souvent et ont comme conséquence visible un vieillissement prématuré au niveau de la peau, une dépigmentation, des phénomènes d'inflammation. Ils peuvent même parfois aboutir à des processus de cancérisation et à des phénomènes de nécrose des tissus.

Il est donc important de mettre au point des systèmes capables de lutter activement contre ces agressions et leurs conséquences. La recherche de moyens afin de préserver la peau et les phanères, voire de les protéger contre toutes manifestations susceptibles d'altérer leur bon fonctionnement ou leur aspect, mobilise un très grand nombre de chercheurs, notamment dans le domaine des cosmétiques.

Les professionnels de la santé et de la cosmétique sont donc en permanence à la recherche de principes actifs performants, réellement efficaces et ayant un large spectre d'action. De plus, ces actifs se doivent d'avoir une forte compatibilité dermatologique de façon à ce que chez tous les utilisateurs, même les plus sensibles, il n'y ait aucune réaction d'irritation. Il subsiste donc le besoin de développer des actifs dermatologiques et/ou cosmétiques ayant une très bonne efficacité ainsi qu'un large spectre d'action au niveau de la peau et des phanères.

Or, de manière inattendue et tout à fait surprenante, les inventeurs ont mis en évidence une activité thérapeutique et, plus particulièrement dermatologique et cosmétique, des protéines SIRT et/ou de ces fragments polypeptidiques ou peptidiques. Il a notamment été mis en évidence que ces protéines et/ou ces fragments peptidiques, lorsqu'ils sont appliqués sur la peau, ont une activité remarquable. En effet, ces composés sont capables de protéger la peau et les phanères contre les agressions extérieures. Ils permettent ainsi d'ouvrir de nouvelles perspectives thérapeutiques et cosmétiques.

Les protéines SIRT font partie de la famille des Sirtuines, ce sont des protéines nucléaires, NAD+ dépendantes, jouant un rôle important dans la désacétylation des histones. Les gènes SIR (Silent Information Régulators), codant pour les protéines SIR, ont pour la première fois été décrits chez S. cerevisiae en 1979 *(*Rine J et Al., Genetics 1979*)*. Plus tard, il a été démontré qu'une surexpression de la protéine SIR2P, chez C. elegans, permettait d'augmenter la durée de vie de cet organisme (Tissenbaum et Al., Nature 2001) ; cette étude émettant ainsi l'hypothèse selon laquelle ces protéines seraient liées à la longévité.

La protéine SIRT1 est la sirtuine humaine la mieux caractérisée, interagissant avec de nombreux régulateurs de la transcription. La protéine humaine SIRT1 a été décrite comme étant impliquée dans la régulation de p53 *(*Cheng HL et Al. Proc Natl Acad Sci U S A. 2003*) ;* et, plus récemment, comme un modulateur de la sénescence cellulaire (Langley E et Al., EMBO J. 2002*).* D'autres protéines humaines Sir ont été découvertes (SIRT2, SIRT3, SIRT4-7). La protéine humaine SIRT2 a été peu étudiée, quelques études ont cependant démontré son rôle dans le contrôle de l'activité mitotique (Dryden SC et Al., Mol Cell Biol. 2003*)* ainsi que son implication dans la régulation de la protéine p53 *(*Vaziri H et Al., Cell. 2001)*.* A ce jour, les sirtuines désacétylases sont considérées comme une famille d'enzymes occupant un rôle important dans la régulation de la mort cellulaire et dans son cycle de vie (Porcu M. and Chiarugi A., Trends Pharmacol Sci., 2005*).*

Ainsi, selon un premier aspect, la présente invention a pour objet une composition cosmétique et/ou dermatologique, caractérisée en ce qu'elle contient au moins un fragment peptidique ou polypeptidique de la famille des protéines SIRT dont le nombre d'acides aminés est compris entre 4 et 25, en tant qu'agent actif, seul ou en association avec au moins un autre agent actif, destinée à être appliquée sur la peau, les muqueuses et/ou les phanères.

Selon la présente invention, lesdits fragments peptidiques de protéines de la famille des SIRT sont des fragments peptidiques dont le nombre d'acides aminés est compris entre 4 et 25. Tous ces fragments peptidiques possèdent une activité biologique.

A ce jour, aucune utilisation de fragments de protéines SIRT dans des compositions cosmétiques ou dermatologiques n'a été décrite.

Selon un mode de réalisation actuellement préféré de l'invention, les fragments peptidiques ou polypeptidiques, sont issus d'une sous-famille particulière des protéines SIRT : les protéines SIRT1.

Selon une méthode particulièrement avantageuse de réalisation de l'invention, le peptide appartenant à la famille des SIRT est choisi parmi les peptides correspondants aux séquences ID N° :
(1) Gly Ala Gly Ile Ser Thr
(2) Gly Ile Pro Asp Phe Arg Ser Pro
(3) Gly Ile Pro Asp Phe Arg
(4) Gly Ile Pro Asp Phe Arg Ser
(5) Gly Leu Tyr Asp Asn Leu Glu
(6) Thr Gln Asn Ile Asp Thr Leu

Selon une méthode de réalisation de l'invention tout particulièrement préférée, le fragment peptidique issu de la famille des SIRT possède la séquence ID N°(5), c'est-à-dire la séquence Gly Leu Tyr Asp Asn Leu Glu.

Dans l'invention, le terme "acide aminé" se réfère ici à tout acide organique naturel ou non naturel ayant la formule :

NHR-CR-C(O)-O-

où chaque -R est indépendamment sélectionné entre un hydrogène et un groupement alkyl ayant entre 1 et 12 atomes de carbone. Préférentiellement, au moins un groupement -R de chaque acide aminé est un hydrogène. Par le terme "alkyl", on entend ici une chaîne carbonée pouvant être linéaire ou ramifiée, substituée (mono- ou poly-) ou non-substituée ; saturée, mono-saturée (une double ou triple liaison dans la chaîne) ou poly-insaturée (deux ou plusieurs doubles liaisons, deux ou plusieurs triples liaisons, une ou plusieurs doubles liaisons et une ou plusieurs triples liaisons dans la chaîne).

Le terme « peptide » désigne un enchaînement de deux ou plusieurs acides aminés liés entre eux par des liaisons peptidiques ou par des liaisons peptidiques modifiées ; un polypeptide désignant un peptide de taille plus importante.

Par peptide, il faut entendre le peptide naturel ou synthétique de l'invention tel que décrit ci-dessus ou au moins l'un de ses fragments, qu'il soit obtenu par protéolyse ou de manière synthétique ou encore tout peptide naturel ou synthétique dont la séquence est totalement ou partiellement constituée par la séquence du peptide précédemment décrit.

Il se peut que pour des questions de résistance à la dégradation, il soit nécessaire d'utiliser une forme protégée du peptide selon l'invention. La forme de protection doit évidemment être une forme biologiquement compatible et doit être compatible avec une utilisation dans le domaine des cosmétiques ou de la pharmacie.

De nombreuses formes de protection biologiquement compatibles peuvent être envisagées, elles sont bien connues de l'homme du métier comme, par exemple, l'acylation ou l'acétylation de l'extrémité amino-terminale, ou l'amidation ou l'estérification de l'extrémité carboxy-terminale. Ainsi, l'invention concerne une utilisation telle que définie précédemment caractérisée par le fait que le peptide est sous forme protégée ou non. De préférence, on utilise une protection basée soit sur l'acylation ou l'acétylation de l'extrémité amino-terminale, soit sur l'amidation ou l'estérification de l'extrémité carboxy-terminale, soit encore des deux. Les dérivés d'acides aminés et les dérivés de peptides concernent aussi les acides aminés et les peptides reliés entre eux par une liaison pseudo-peptidique. On entend par "liaison pseudo-peptidique" tous les types de liaisons susceptibles de remplacer les liaisons peptidiques "classiques".

Dans le domaine des acides aminés, la géométrie des molécules est telle qu'elles peuvent théoriquement se présenter sous la forme d'isomères optiques différents. Il existe, en effet, une conformation moléculaire de l'acide aminé (AA) telle qu'elle dévie à droite le plan de polarisation de la lumière (conformation dextrogyre ou D-aa), et une conformation moléculaire de l'acide aminé (aa) telle qu'elle dévie à gauche le plan de polarisation de la lumière (conformation lévogyre ou L-aa). La nature n'a retenu pour les acides aminés naturels que la conformation lévogyre. En conséquence, un peptide d'origine naturelle ne sera constitué que d'acides aminés de type L-aa. Cependant la synthèse chimique en laboratoire permet de préparer des acides aminés ayant les deux conformations possibles. A partir de ce matériel de base, il est ainsi possible d'incorporer lors de la synthèse de peptide aussi bien des acides aminés sous forme d'isomères optiques dextrogyre ou lévogyre. Ainsi, les acides aminés constituant le peptide selon l'invention peuvent être sous configuration L- et D- ; de manière préférentielle, les acides aminés sont sous forme L. Le peptide selon l'invention peut donc être sous forme L-, D- ou DL-.

Les peptides selon l'invention peuvent être préparés par toutes méthodes appropriées, ainsi les peptides peuvent être des peptides isolés à partir de peptides et de protéines existant naturellement, des peptides recombinants, des peptides de synthèse, ou encore des peptides produits par une combinaison de ces méthodes. Bien entendu, les méthodes afin de préparer les peptides selon l'invention sont bien connues de l'homme du métier. Ainsi, le peptide selon l'invention peut être un peptide d'origine naturelle ou synthétique. Préférentiellement selon l'invention, le peptide est obtenu par synthèse chimique.

Selon un mode de réalisation avantageux de l'invention, les peptides précités, selon l'invention, sont préalablement solubilisés dans un ou plusieurs solvants cosmétiquement ou pharmaceutiquement acceptables, classiquement utilisés par l'homme du métier, comme l'eau, l'éthanol, le propylène glycol, le butylène glycol, le dipropylène glycol, les diglycols éthoxylés ou propoxylés, les polyols cycliques, la vaseline, une huile végétale ou tout mélange de ces solvants.

Selon encore un autre mode de réalisation avantageux de l'invention, les peptides précités sont préalablement solubilisés dans un vecteur cosmétique ou pharmaceutique comme les liposomes ou adsorbés sur des polymères organiques poudreux, des supports minéraux comme les talcs et bentonites, et plus généralement solubilisés dans, ou fixés sur, tout vecteur cosmétiquement ou pharmaceutiquement acceptable.

Il est bien entendu que le peptide selon l'invention peut être utilisé seul ou bien en association avec au moins un autre agent actif, dans ou pour la préparation d'une composition cosmétique et/ou dermatologique et/ou pharmaceutique.

Un aspect essentiel de l'invention est une composition contenant au moins un fragment peptidique, peptide de la famille des protéines SIRT dont le nombre d'acides aminés est compris entre 4 et 25, à usage topique, destinée à être utilisée, notamment, pour le soin de la peau et des phanères.

Lesdits peptides sont aussi avantageusement utilisés afin de protéger les substrats kératiniques, et plus particulièrement afin de protéger la peau et/ou les phanères contre tous les types d'agressions extérieures. Selon une méthode de réalisation actuellement préférée de l'invention, ledit peptide est choisi parmi le groupe de peptides correspondants aux séquences ID N°(1) à N°(6).

L'invention a, en outre, pour objet une composition contenant au moins un composé tel que défini précédemment, destinée à être utilisée pour traiter de manière curative et/ou préventive les manifestations cutanées du vieillissement, mais aussi, d'une manière plus générale, pour améliorer l'aspect de la peau et/ou des phanères. Plus généralement le composé actif selon l'invention aura une activité efficace dans tous les soins de la peau et/ou des phanères. Par les soins de la peau et/ou des phanères, on entend toutes les actions destinées à conserver ou à rétablir un bon fonctionnement de la peau et/ou des phanères ou encore tout moyen qui sert à préserver ou à améliorer leur apparence et/ou leur aspect. Ainsi le soin inclut l'hydratation, l'apaisement, la protection contre tous types d'agressions, notamment la protection solaire, la lutte et la prévention des manifestations cutanées du vieillissement.

Par manifestations cutanées du vieillissement on entend toutes modifications de l'aspect extérieur de la peau dues au vieillissement comme, par exemple, les rides et ridules, la peau flétrie, la peau molle, la peau amincie, le manque d'élasticité et/ou de tonus de la peau, la peau terne et sans éclat mais également toutes modifications internes de la peau qui ne se traduisent pas systématiquement par un aspect extérieur modifié comme, par exemple, toutes dégradations internes de la peau consécutives à une exposition aux rayonnements ultra-violets. Par l'expression "améliorer l'aspect de la peau", on entend tous les phénomènes qui sont susceptibles d'avoir pour conséquence une amélioration visuelle de l'état de la peau. La peau présentera une meilleure apparence ; elle sera, par exemple, beaucoup plus belle, ferme et/ou lisse. Toutes les petites imperfections seront diminuées ou supprimées. L'aspect papyracé de la peau sera, par exemple, atténué.

L'utilisation de cet agent actif, ou d'une composition le contenant, va permettre aux substrats kératiniques d'être protégés et de mieux résister au stress que produit sur eux l'environnement. Plus précisément, la présente invention vise une composition contenant au moins un composé, tel que défini précédemment, en tant qu'agent actif destinée à être utilisée pour protéger la peau et/ou les phanères contre tous les types d'agressions extérieures. On entend par le terme "agression extérieure" les agressions que peut produire l'environnement. Ces agressions peuvent être d'origine chimique, physique, biologique ou thermique. A titre d'exemple, on peut citer des agressions telles que la pollution, les UV, les frottements, l'eau à forte concentration de calcaire, les variations de température ou encore les produits à caractère irritant tels que les tensioactifs, les conservateurs ou les parfums.

Par ailleurs, les peptides selon l'invention, ou la composition les contenant, ont des effets anti-inflammatoires et anti-irritants. L'utilisation des propriétés de cet agent cosmétique permet donc d'avoir une peau plus protégée et nettement moins sensible aux diverses agressions qu'elle peut rencontrer. La peau est ainsi apaisée.

De plus, les composés selon l'invention stimulent le fonctionnement métabolique des cellules, et notamment des cellules de la peau, ils permettent ainsi d'augmenter la synthèse de protéines essentielles à son fonctionnement, notamment en augmentant la synthèse de protéines constitutives de la matrice extra-cellulaire. Les peptides selon l'invention, ou la composition les contenant, possèdent ainsi une action positive sur la régénération tissulaire et sur la cicatrisation.

Les peptides, issus de la famille des protéines SIRT1, précédemment définis, sont ainsi utilisés pour la fabrication d'une composition pharmaceutique et/ou cosmétique, à usage topique. Ils seront utilisés d'une manière plus générale afin de traiter les affections dermatologiques. Par affections dermatologiques, on entend toutes les maladies affectant la peau et ayant ou non des conséquences visibles. A ce titre, on peut citer par exemple : des désordres liés à des problèmes de différenciations et de proliférations cellulaires, des problèmes liés à la kératinisation, des troubles inflammatoires ou allergiques, des troubles liés aux fonctions sébacées, des proliférations dermiques ou épidermiques (bénignes ou malignes) ; des désordres cutanés dus à une exposition aux rayonnements U.V., des pathologies associées au vieillissement chronologique ou actinique.

Ainsi, selon un autre aspect, l'invention concerne une composition contenant les peptides selon l'invention, tels que décrits précédemment, destinée à être utilisée dans le traitement des affections dermiques.

L'invention concerne une composition cosmétique et/ou dermatologique et/ou pharmaceutique caractérisée en ce qu'elle contient, dans un milieu acceptable, comme principe actif, des fragments polypeptidiques ou peptidiques de protéines de la famille des SIRT, ou peptides tels que définis précédemment. D'une manière avantageuse la composition selon l'invention contient un ou plusieurs peptides choisis parmi les peptides correspondants aux séquences ID N° (1) à ID N° (6). Selon une réalisation particulièrement avantageuse de l'invention, la composition contient le peptide de séquence ID N°(5), c'est-à-dire la séquence Gly Leu Tyr Asp Asn Leu Glu.

Dans la composition selon l'invention, le peptide peut être un mélange de dérivés peptidiques et/ou constitué de dérivés d'acides aminés. Il est bien entendu que le peptide selon l'invention peut être utilisé seul ou en association avec au moins un autre agent actif.

La composition contenant le peptide selon l'invention peut être une composition cosmétique ou dermatologique ou pharmaceutique. Préférentiellement selon l'invention, la composition est une composition cosmétique, car elle est destinée à améliorer l'aspect et les performances cutanées générales de l'individu qui en fait usage. La composition selon l'invention est préférentiellement une composition cosmétique et/ou dermatologique adaptée à l'administration par voie topique cutanée comprenant un milieu cosmétiquement ou pharmaceutiquement acceptable.

Il est bien évident que l'invention s'adresse aux mammifères en général et plus particulièrement aux êtres humains. La quantité efficace de principe actif correspond à la quantité nécessaire afin d'obtenir le résultat désiré.

Selon un mode de réalisation avantageux de l'invention, le peptide précité est présent dans les compositions de l'invention à une concentration comprise entre 0,005 et 500 ppm (parties par million) environ, et préférentiellement à une concentration comprise entre 0,1 et 50 ppm environ par rapport au poids total de la composition finale.

Quelle que soit la forme de l'invention, la composition selon l'invention peut être injectée ou appliquée sur la peau (sur toute zone cutanée du corps), les cheveux, les ongles ou les muqueuses. Selon le mode d'administration, la composition selon l'invention peut se présenter sous toutes les formes galéniques normalement utilisées.

Préférentiellement, les compositions selon la présente invention se présenteront sous une forme galénique adaptée à l'administration par voie topique cutanée, et couvrent toutes les formes cosmétiques ou dermatologiques. Ces compositions doivent donc contenir un milieu cosmétiquement acceptable, c'est-à-dire compatible avec la peau, les muqueuses et les phanères.

Ces compositions pourront notamment se présenter sous forme d'une solution aqueuse, hydroalcoolique ou huileuse ; d'une émulsion huile-dans-eau, eau-dans-huile ou émulsions multiples ; elles peuvent aussi se présenter sous forme de crèmes, de suspensions, ou encore de poudres, adaptées à une application sur la peau, les muqueuses, les lèvres et/ou les phanères. Ces compositions peuvent être plus ou moins fluides et avoir l'aspect d'une crème, d'une lotion, d'un lait, d'un sérum, d'une pommade, d'un gel, d'une pâte ou d'une mousse. Elles peuvent aussi se présenter sous forme solide, comme un stick ou être appliquées sur la peau sous forme d'aérosol. Elles peuvent être utilisées comme produit de soin et/ou comme produit de maquillage de la peau.

Ces compositions comprennent, en outre, tout additif communément utilisé dans le domaine d'application envisagé ainsi que les adjuvants nécessaires à leur formulation, tels que des solvants, des épaississants, des diluants, des anti-oxydants, des colorants, des filtres solaires, des agents auto-bronzants, des pigments, des charges, des conservateurs, des parfums, des absorbeurs d'odeur, des actifs cosmétiques ou pharmaceutiques, des huiles essentielles, des vitamines, des acides gras essentiels, des tensioactifs, des polymères filmogènes, etc.

Dans tous les cas, l'homme du métier veillera à ce que ces adjuvants ainsi que leurs proportions soient choisis de telle manière à ne pas nuire aux propriétés avantageuses recherchées de la composition selon l'invention. Ces adjuvants peuvent, par exemple, correspondre de 0,01 à 20 % du poids total de la composition. Lorsque la composition de l'invention est une émulsion, la phase grasse peut représenter de 5 à 80 % en poids et de préférence de 5 à 50 % en poids par rapport au poids total de la composition. Les émulsionnants et co-émulsionnants utilisés dans la composition seront choisis parmi ceux classiquement utilisés dans le domaine considéré. Par exemple, ils peuvent être utilisés en une proportion allant de 0,3 à 30 % en poids, par rapport au poids total de la composition.

Bien entendu, l'homme du métier veillera à choisir les éventuels composés complémentaires, actifs ou non-actifs, et/ou leurs quantités, de telle sorte que les propriétés avantageuses du mélange ne soient pas, ou sensiblement pas, altérées par l'adjonction envisagée.

Les compositions selon l'invention trouvent une application notamment comme compositions cosmétiques ou pharmaceutiques pour la peau, les muqueuses et/ou les semi-muqueuses. Elles trouvent une application en tant que produit de protection et/ou de soin de la peau, ou encore en tant que composition anti-rides et/ou anti-âge.

D'autres avantages et caractéristiques de l'invention apparaîtront mieux à la lecture des exemples donnés à titre illustratif et non limitatif.

### Exemple : Préparation de compositions.

Les quantités indiquées sont données en pourcentage de poids.

### 1 - Crème de soin antirides:

| **Noms commerciaux** | **Noms INCI** | **% massique** |
|---|---|---|
| *PHASE A* | | |
| Montanov 68 | Cetearyl Alcohol (and) Cetearyl Glucoside | 6.00 |
| Squalane | Squalane | 3.00 |
| Cetiol SB 45 | Butyrospermum Parkii ( Shea Butter) | 2.00 |
| Waglinol 250 | Cetearyl Ethylhexanoate | 3.00 |
| Amerchol L- 101 | Mineral Oil (and) Lanolin Alcohol | 2.00 |
| Abil 350 | Dimethicone | 1.50 |
| BHT | BHT | 0.01 |
| *PHASE B* | | |
| Eau déminéralisée | Aqua (Water) | qsp |
| Butylene Glycol | Butylene Glycol | 2.00 |
| Glucam E10 | Methyl Gluceth-10 | 1.00 |
| Allantoin | Allantoin | 0.15 |
| Carbopol Ultrez 10 | Carbomer | 0.20 |
| *PHASE C* | | |
| Huile d'Avocat | Persea Gratissima (Avocado) Oil | 1.25 |
| Phenonip | Phenoxyethanol (and) | 0.75 |
| | Methylparaben (and) Ethylparaben (and) Butylparaben (and) | |
| | Propylparaben (and) Isobutylparaben | |
| *PHASE D* | | |
| TEA | Triethanolamine | 0.18 |
| *PHASE E* | | |
| Peptide selon l'invention | | 2 ppm |
| Parfum | Parfum (Fragrance) | qsp |
| Colorant | | qsp |

Les constituants de la phase A et de la phase B sont chauffés séparément à une température comprise entre 65°C et 70°C, la phase C est incorporée, puis la phase A est émulsionnée dans la phase B. Le Carbomer est neutralisé avec la phase D à une température aux alentours de 45°C. La phase E est ensuite additionnée sous agitation et le refroidissement est poursuivi jusqu'à 25°C.

### 2 - Lait corporel anti-âge:

| ***Noms commerciaux*** | ***Noms INCI*** | ***% massique*** |
|---|---|---|
| *PHASE A* | | |
| Eau déminéralisée | Aqua (Water ) | qsp |
| Carbopol EDT 2020 | Acrylates/C10-30 Alkylacrylate Crosspolymer | 0.10 |
| Glycerine | Glycerin | 1.20 |
| EDTA | Trisodium EDTA | 0.65 |
| Propylene Glycol | Propylene Glycol | 1.50 |
| *PHASE B* | | |
| Miglyol 812 | Caprylic/Capric Triglyceride | 2.50 |
| Amerchol L 101 | Mineral Oil (and) Lanolin Alcohol | 2.00 |
| Squalane | Squalane | 1.50 |
| Cetiol SN | Cetearyl Isononanoate | 2.00 |
| Stearine TP | Stearic Acid | 2.00 |
| Tegin | Glyceryl Stearate SE | 3.00 |
| Lanette 16 | Cetyl Alcohol | 0.20 |
| Dow Corning 200 Fluid | Dimethicone | 0.50 |
| Phenonip | Phenoxyethanol (and) Methylparaben (and) Ethylparaben (and) Butylparaben (and) Propylparaben (and) Isobutylparaben | 0.70 |
| *PHASE C* | | |
| TEA | Triethanolamine | 0.08 |
| *PHASE D* | | |
| Peptide selon l'invention | | 0.5 ppm |
| Parfum | Parfum (Fragrance) | qsp |
| Colorant | | qsp |

Les constituants de la phase A et de la phase B sont chauffés séparément à une température comprise entre 70°C et 75°C. La phase B est émulsionnée dans A sous agitation « Staro ». Après un refroidissement jusqu'à 50°C, le mélange est neutralisé avec la phase C. La phase D est ensuite additionnée lorsque la température se situe en dessous de 40°C. Le refroidissement est poursuivi jusqu'à 25°C sous agitation lente.

### 3 - Crème protection solaire:

| ***Noms commerciaux*** | ***Noms INCI*** | ***% massique*** |
|---|---|---|
| *PHASE A* | | |
| Eau déminéralisée | Aqua (Water) | qsp |
| Pemulen TR1 | Acrylates/C10-30 Alkyl Acrylate Crosspolymer | 0.40 |
| Glycerine | Glycerin | 3.00 |
| Nipastat Sodium | Sodium Methylparaben (and) Sodium Ethylparaben (and) Sodium Butyl paraben (and) Sodium Propylparaben (and) Sodium Isobutylparaben | 0.15 |
| *PHASE B* | | |
| Parsol MCX | Ethylhexyl Methoxycinnamate | 7.50 |
| Eusolex 4360 | Benzophenone-3 | 3.00 |
| Parsol 1789 | Butyl Methoxydibenzoylmethane | 2.00 |
| Myritol 318 | Caprylic/Capric Triglyceride | 4.00 |
| Emulgade SEV | Hydrogenated Palm Glycerides (and) Ceteareth-20 (and) Ceteareth-12 (and) Cetearyl Alcohol | 5.00 |
| Propylparaben | Propylparaben | 0.15 |
| Nacol 16-98 | Cetyl Alcohol | 1.00 |
| *PHASE C* | | |
| TEA | Triethanolamine | 0.20 |
| *PHASE D* | | |
| Peptide selon l'invention | | 1 ppm |
| Parfum | Parfum (Fragrance) | qsp |
| Colorant | | qsp |

Les constituants de la phase A et de la phase B sont chauffés séparément à une température comprise entre 70°C et 75°C. La phase B est émulsionnée dans A sous agitation. La phase C est ajoutée, à 45°C, en augmentant l'agitation. La phase D est ensuite additionnée lorsque la température se situe en dessous de 40°C. Le refroidissement est poursuivi jusqu'à 25°C sous vive agitation.

## Revendications

1. Composition cosmétique et/ou dermatologique, **caractérisée en ce qu'**elle contient au moins un fragment peptidique ou polypeptidique de la famille des protéines SIRT dont le nombre d'acides aminés est compris entre 4 et 25, en tant qu'agent actif, seul ou en association avec au moins un autre agent actif, destinée à être appliquée sur la peau, les muqueuses et/ou les phanères.

2. Composition selon la revendication 1, **caractérisée en ce que** le fragment est issu des protéines SIRT1.

3. Composition selon la revendication 1 ou 2, **caractérisée en ce que** le fragment est choisi parmi les peptides correspondants aux séquences ID N°.
(1) Gly Ala Gly Ile Ser Thr
(2) Gly Ile Pro Asp Phe Arg Ser Pro
(3) Gly Ile Pro Asp Phe Arg
(4) Gly Ile Pro Asp Phe Arg Ser
(5) Gly Leu Tyr Asp Asn Leu Glu
(6) Thr Gln Asn Ile Asp Thr Leu

4. Composition selon l'une quelconque des revendications précédentes, **caractérisée en ce que** le fragment possède la séquence ID N° (5), c'est-à-dire la séquence Gly Leu Tyr Asp Asn Leu Glu.

5. Composition selon l'une quelconque des revendications 1 à 3, **caractérisée en ce que** le fragment possède la séquence ID N° (6), c'est-à-dire la séquence Thr Gln Asn Ile Asp Thr Leu.

6. Composition selon l'une quelconque des revendications précédentes, **caractérisée en ce que** le fragment est choisi parmi les peptides ou polypeptides dont au moins un groupement fonctionnel est protégé par un groupement protecteur, ce groupement protecteur étant soit une acylation ou une acétylation de l'extrémité amino-terminale, soit sur une amidation ou une estérification de l'extrémité carboxy-terminale, soit les deux.

7. Composition selon l'une quelconque des revendications précédentes, **caractérisée en ce que** le fragment est présent, dans la composition, à une concentration comprise entre 0,05 et 500 ppm environ, et préférentiellement à une concentration comprise entre 0,1 et 50 ppm environ par rapport au poids total de la composition finale.

8. Composition selon l'une quelconque des revendications précédentes, **caractérisée en ce que** le fragment est préalablement solubilisé dans un ou plusieurs solvants cosmétiquement ou pharmaceutiquement acceptables comme l'eau, l'éthanol, le propylène glycol, le butylène glycol, le dipropylène glycol, les diglycols éthoxylés ou propoxylés, les polyols cycliques, la vaseline, une huile végétale ou tout mélange de ces solvants.

9. Composition selon l'une quelconque des revendications précédentes, **caractérisée en ce que** le fragment est préalablement solubilisé dans un vecteur cosmétique ou pharmaceutique comme les liposomes ou adsorbés sur des polymères organiques poudreux, des supports minéraux comme les talcs et bentonites, et plus généralement solubilisés dans, ou fixés sur, tout vecteur cosmétiquement ou pharmaceutiquement acceptable.

10. Composition selon l'une quelconque des revendications 1 à 9, contenant une quantité efficace d'au moins un fragment actif, destinée à être utilisée pour les soins de la peau et/ou des phanères.

11. Composition selon l'une quelconque des revendications 1 à 9, contenant une quantité efficace d'au moins un fragment actif, destinée à être utilisée pour lutter de manière curative et/ou préventive contre les manifestations du vieillissement cutané, et/ou améliorer l'aspect de la peau et/ou des phanères.

12. Composition selon l'une quelconque des revendications 1 à 9, contenant une quantité efficace d'au moins un fragment actif, destinée à être utilisée pour protéger la peau et/ou les phanères contre tous les types d'agressions extérieures.

13. Composition selon l'une quelconque des revendications 1 à 9, contenant une quantité efficace d'au moins un fragment actif, destinée à être utilisée pour diminuer et/ou prévenir les réactions inflammatoires et/ou irritantes cutanées.

14. Composition selon l'une quelconque des revendications 1 à 9, contenant une quantité efficace d'au moins un fragment actif, destinée à être utilisée dans le traitement des affections dermiques.

15. Composition selon l'une quelconque des revendications 1 à 9, **caractérisée en ce qu'**elle se présente sous la forme d'une composition cosmétique et/ou dermatologique adaptée à l'administration par voie topique cutanée comprenant un milieu cosmétiquement ou pharmaceutiquement acceptable.

## Patentansprüche

1. Kosmetische und/oder dermatologische Zusammensetzung, **dadurch gekennzeichnet, dass** sie mindestens ein Peptid- oder Polypeptid-Fragment der Familie der SIRT-Proteine, bei denen die Anzahl von Aminosäuren zwischen 4 und 25 liegt, als Wirkstoff enthält, alleine oder in Verbindung mit mindestens einem anderen Wirkstoff, die ausgelegt ist, um auf die Haut, der Schleimhäute und/oder die Hautanhangsgebilde aufgebracht zu werden.

2. Zusammensetzung nach Anspruch 1, **dadurch gekennzeichnet, dass** das Fragment aus den SIRT1-Proteinen stammt.

3. Zusammensetzung nach Anspruch 1 oder 2, **dadurch gekennzeichnet, dass** das Fragment ausgewählt ist aus den Peptiden, die den folgenden Sequenz ID Nr. entsprechen:
(1) Gly Ala Gly Ile Ser Thr
(2) Gly Ile Pro Asp Phe Arg Ser Pro
(3) Gly Ile Pro Asp Phe Arg
(4) Gly Ile Pro Asp Phe Arg Ser
(5) Gly Leu Tyr Asp Asn Leu Glu
(6) Thr Gln Asn Ile Asp Thr Leu

4. Zusammensetzung nach einem der vorhergehenden Ansprüche, **dadurch gekennzeichnet, dass** das Fragment die Sequenz ID Nr. (5) aufweist, d.h. die Sequenz Gly Leu Tyr Asp Asn Leu Glu.

5. Zusammensetzung nach einem der Ansprüche 1 bis 3, **dadurch gekennzeichnet, dass** das Fragment die Sequenz ID Nr. (6) aufweist, d.h. die Sequenz Thr Gln Asn Ile Asp Thr Leu.

6. Zusammensetzung nach einem der vorhergehenden Ansprüche, **dadurch gekennzeichnet, dass** das Fragment ausgewählt ist aus den Peptiden oder Polypeptiden, bei denen mindestens eine funktionelle Gruppe von einer Schutzgruppe geschützt wird, wobei diese Schutzgruppe entweder eine Acylierung oder eine Acetylierung des amino-terminalen Endes ist oder eine Amidbildung oder eine Veresterung des carboxy-terminalen Endes oder beides ist.

7. Zusammensetzung nach einem der vorhergehenden Ansprüche, **dadurch gekennzeichnet, dass** das Fragment in der Zusammensetzung in einer Konzentration, die ungefähr zwischen 0,05 und 500 ppm und vorzugsweise in einer Konzentration, die ungefähr zwischen 0,1 und 50 ppm mit Bezug auf das Gesamtgewicht der endgültigen Zusammensetzung liegt, vorhanden ist.

8. Zusammensetzung nach einem der vorhergehenden Ansprüche, **dadurch gekennzeichnet, dass** das Fragment zuvor in einem oder in mehreren Lösemitteln solubilisiert wird, die kosmetisch oder pharmazeutisch annehmbar sind, wie z.B. Wasser, Ethanol, Propylenglycol, Butylenglycol, Dipropylenglycol, ethoxylierten oder propoxylierten Diglykolen, cyclischen Polyolen, Vaselin, einem pflanzlichen Öl oder jeder Mischung dieser Lösemittel.

9. Zusammensetzung nach einem der vorhergehenden Ansprüche, **dadurch gekennzeichnet, dass** das Fragment vorher in einem kosmetischen oder pharmazeutischen Vektor solubilisiert wird, wie z.B. den Liposomen, oder adsorbiert auf pulverförmigen organischen Polymeren, mineralischen Trägern wie z.B. Talkmineralien und Bentoniten und insbesondere solubilisiert in oder fixiert auf in jedem kosmetisch oder pharmazeutisch annehmbaren Vektor.

10. Zusammensetzung nach einem der Ansprüche 1 bis 9, enthaltend eine wirksame Menge mindestens eines aktiven Fragments, das ausgelegt ist, um für die Pflege der Haut und/oder der Hautanhangsgebilde verwendet zu werden.

11. Zusammensetzung nach einem der Ansprüche 1 bis 9, enthaltend eine wirksame Menge mindestens eines aktiven Fragments, die ausgelegt ist, um dazu verwendet zu werden, auf heilende und/oder vorbeugende Weise gegen die Anzeichen der Alterung der Haut zu kämpfen und/oder das Aussehen der Haut und/oder der Hautanhangsgebilde zu verbessern.

12. Zusammensetzung nach einem der Ansprüche 1 bis 9, enthaltend eine wirksame Menge mindestens eines aktiven Fragments, das ausgelegt ist, um verwendet zu werden, um die Haut und/oder die Hautanhangsgebilde gegen alle Arten von äußeren Aggressionen zu schützen.

13. Zusammensetzung nach einem der Ansprüche 1 bis 9, enthaltend eine wirksame Menge mindestens eines aktiven Fragments, das ausgelegt ist, um verwendet zu werden, um die entzündlichen und/oder irritierenden Reaktionen der Haut zu verringern und/oder ihnen vorzubeugen.

14. Zusammensetzung nach einem der Ansprüche 1 bis 9, enthaltend eine wirksame Menge mindestens eines aktiven Fragments, das ausgelegt ist, um bei der Behandlung von Hauterkrankungen verwendet zu werden.

15. Zusammensetzung nach einem der Ansprüche 1 bis 9, **dadurch gekennzeichnet, dass** sie die Form einer kosmetischen und/oder dermatologischen Zusammensetzung aufweist, die ausgelegt ist, um auf topischem kutanem Weg verabreicht zu werden, umfassend ein kosmetisch oder pharmazeutisch annehmbares Medium.

## Claims

1. Cosmetic and/or dermatological composition, **characterised in that** it contains at least one peptide or polypeptide fragment in the family of SIRT proteins, the number of amino acids of which is between 4 and 25, as an active agent, alone or in association with at least one other active agent, intended to be applied to the skin, the mucous membranes and/or the keratinous appendages.

2. Composition according to claim 1, **characterised in that** the fragment issues from SIRT1 proteins.

3. Composition according to Claim 1 or 2, **characterised in that** the fragment is chosen from the peptides corresponding to the sequences ID No.:
(1) Gly Ala Gly Ile Ser Thr
(2) Gly Ile Pro Asp Phe Arg Ser Pro
(3) Gly Ile Pro Asp Phe Arg
(4) Gly Ile Pro Asp Phe Arg Ser
(5) Gly Leu Tyr Asp Asn Leu Glu
(6) Thr Gln Asn Ile Asp Thr Leu

4. Composition according to any one of the preceding claims, **characterised in that** the fragment has the sequence ID No. (5), that is to say the sequence Gly Leu Tyr Asp Asn Leu Glu.

5. Composition according to any one of claims 1 to 3, **characterised in that** the fragment has the sequence ID No. (6), that is to say the sequence Thr Gln Asn Ile Asp Thr Leu.

6. Composition according to any one of the preceding claims, **characterised in that** the fragment is chosen from the peptides or polypeptides, at least one functional group of which is protected by a protective group, this protective group being either an acylation or an acetylation of the aminoterminal end, or an amidation or an esterification of the carboxyterminal end, or both.

7. Composition according to any one of the preceding claims, **characterised in that** the fragment is present, in the composition, at a concentration of between 0.05 and 500 ppm approximately, and preferentially at a concentration of between 0.1 and 50 ppm approximately with respect to the total weight of the final composition.

8. Composition according to any one of the preceding claims, **characterised in that** the fragment is previously solubilised in one or more cosmetically or pharmaceutically acceptable solvents such as water, ethanol, propylene glycol, butylene glycol, dipropylene glycol, ethoxylated or propoxylated glycols, cyclic polyols, Vaseline, a plant oil or any mixture of these solvents.

9. Composition according to any one of the preceding claims, **characterised in that** the fragment is previously solubilised in a cosmetic or pharmaceutical carrier such as lyposomes or adsorbed on organic polymers in powder form, mineral carriers such as talcs and bentonites, and more generally solubilised in or fixed to any cosmetically or pharmaceutically acceptable carrier.

10. Composition according to any one of claims 1 to 9, containing an effective quantity of at least one active fragment, intended to be used for care of the skin and/or keratinous appendages.

11. Composition according to any one of claims 1 to 9, containing an effective quantity of at least one active fragment, intended to be used for combating, curatively and/or preventatively, manifestations of skin ageing, and/or improving the appearance of the skin and/or keratinous appendages.

12. Composition according to any one of claims 1 to 9, containing an effective quantity of at least one active fragment, intended to be used for protecting the skin and/or the keratinous appendages against all types of external attacks.

13. Composition according to any one of claims 1 to 9, containing an effective quantity of at least one active fragment, intended to be used for reducing and/or preventing inflammatory and/or irritating skin reactions.

14. Composition according to any one of claims 1 to 9, containing an effective quantity of at least one active fragment, intended to be used in the treatment of dermal afflictions.

15. Composition according to any one of claims 1 to 9, **characterised in that** it is in the form of a cosmetic and/or dermatological composition suitable for topical skin administration, comprising a cosmetically or pharmaceutically acceptable medium.
